(19) Europäisches Patentamt
European Patent Office
Office européen des brevets

(11) Publication number: **0 387 802 A2**

(12) **EUROPEAN PATENT APPLICATION**

(21) Application number: 90104742.3

(22) Date of filing: 13.03.90

(51) Int. Cl.5: C07D 471/04, C07D 519/00, A61K 31/44, A61K 31/55, //(C07D471/04,221:00,221:00), (C07D519/00,487:00,471:00)

(30) Priority: 13.03.89 US 322733
05.09.89 US 402418

(43) Date of publication of application:
19.09.90 Bulletin 90/38

(84) Designated Contracting States:
AT BE CH DE DK ES FR GB GR IT LI LU NL SE

(71) Applicant: BRISTOL-MYERS SQUIBB COMPANY
345 Park Avenue
New York, N.Y. 10154(US)

(72) Inventor: DiCesare, Pierre
6 ter, rue de Bonneuil
F-77110 Meaux(FR)
Inventor: Bouzard, Daniel
11, Rue Amiati
F-95130, Franconville(FR)
Inventor: Kiechel, Jean-René
54 Avenue Auguste Renoir
F-92500 Rueil Malmaison(FR)

(74) Representative: Kinzebach, Werner, Dr. et al
Patentanwälte Reitstötter, Kinzebach und Partner Sternwartstrasse 4 Postfach 86 06 49
D-8000 München 86(DE)

(54) **5-Substituted-1,4-dihydro-4-oxonaphthyridine-3-carboxylate antibacterial agents.**

(57) The invention is concerned with naphthyridine compounds which are substituted in the 7-position with various nitrogen heterocyclo groups and at the 1,3,4,5 and 6-positions with certain other groups. These compounds are antibacterial agents and are suitable for the treatment of infections.

EP 0 387 802 A2

## 5-Substituted-1,4-dihydro-4-oxo-naphthyridine-3-carboxylate Antibacterial Agents

### Field of the Invention

This invention is concerned with naphthyridine compounds which are substituted in the 7-position with various nitrogen heterocyclo groups, and at the 1,3,4,5 and 6-positions with certain other groups. They are classified variously in Class/Subclass 546/123, 544/586, 544/349, 544/363, and perhaps others. These compounds have drug and bio-affecting properties.

### Summary of the Invention

The present invention concerns compounds of Formula I, a process for their prepartion, a process for preparing intermediates adapted for preparation of these compounds, a process for treating infectious diseases with these compounds, and pharmaceutical compositions containing these compounds which are in suitable form for administration to mammals.

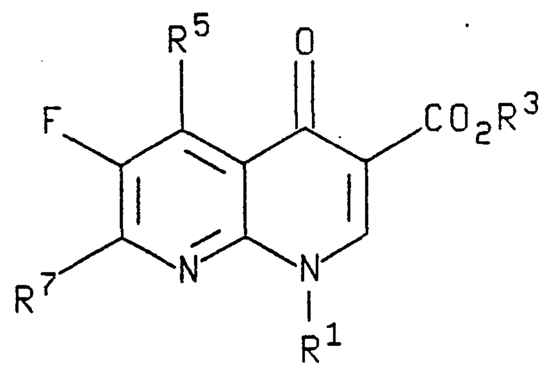

(I)

In the foregoing formula, symbols $R^1$, $R^3$, $R^5$, and $R^7$ have the following meanings:

$R^1$ is selected from cycloalkyl having 3 to 6 carbon atoms, cycloalkylalkyl having from 3 to 7 carbon atoms including 3 to 6 ring members each having up to 3 substituents selected from methyl and fluorine, lower alkyl having 2 to 6 carbon atoms, lower fluoroalkyl having 2 to 6 carbon atoms and 1 to 3 fluorine atoms, and phenyl or substituted phenyl having 1 or 2 fluorine or tri-fluoromethyl substituents;

$R^3$ is hydrogen, lower alkyl having 1 to 4 carbon atoms, a physiologically hydrolyzable ester group, or a carboxy-protecting group of the readily removable type conventionally used in organic synthesis;

$R^5$ is selected from lower alkyl having 1 to 6 carbon atoms, lower alkenyl having 2 to 6 carbon atoms, cycloalkyl having 3 to 6 carbon atoms, cycloalkylalkyl having 4 to 7 carbon atoms, aryl having 6 to 10 carbon atoms, and aralkyl having 7 to 11 carbon atoms;

$R^7$ is a nitrogen heterocycle having one or two rings and 4 to 9 ring members and up to two additional heteroatoms selected from O, S, and N, said heterocycle being covalently attached as shown through a ring-N atom thereof which heterocycle may be unsubstituted or substituted by 1 or 2 substituents having up to 6 carbon atoms and selected from alkyl, hydroxyalkyl, hydroxy, chlorine, fluorine, amino, alkylamino, dialkylamino, aminoalkyl, alkylaminoalkyl, dialkylaminoalkyl, carboxy, carboxyalkyl, and carboxamido,

and the pharmaceutically acceptable acid addition salts thereof, and the pharmaceutically acceptable metal and amine salts of those compounds wherein $R^3$ is H.

The invention also includes the pharmaceutically acceptable metal and amine salts of those compounds of Formula I in which $R^3$ is hydrogen. The acid addition salts of the compounds of Formula I, and particularly those which are pharmaceutically acceptable, are part of the invention. The pharmaceutically acceptable salts referred to above are those which have chemical, and physical properties which are compatible with the preparation of pharmaceutical dosage forms, and which have toxicological properties compatible with the administration of effective doses thereof to mammals in antiinfectively effective doses.

## Background of the Invention

The naphthyridine-3-carboxylic acid antibacterial agents make up a class of compounds which have been extensively described in the literature. This literature includes both patents and publications. A literature and patent summary appears in PCT application Publication No. WO88/0627 published April 21, 1988. More recently a review entitled "Structure Activity Relationships of the Fluoro Quinolones" was published by D. T. W. Chu and P. B. Fernandes in Antimicrobial Agents and Chemotherapy, February, 1989, pages 131-135. Refer particularly to the structural formulas on page 132.

The compounds of the present invention, as is reflected by the foregoing summary, are differentiated structurally from prior antibacterial naphthyridine-3-carboxylic acids by the presence of a carbon attached substituent in the 5-position. A search of the literature has revealed that no antibacterial naphthyridine carboxylic acid which has the combination of a nitrogen attached substituent in the 7-position and a carbon attached substituent in the 5-position has been described prèviously. Napththyridine-3-carboxylic acid antibacterial agents having a nitrogen, oxygen, or sulfur attached substituent, or a halogen atom in the 5-position, have been described. Such compounds having an amino, alkylamino, substituted alkylamino, dialkylamino, alkoxyamino, hydroxy, alkoxy, phenylthio, mercapto, hydrazino, hydroxylamino, methoxyamino, or chloro group in the 5-position have been described in published European Patent Application 284935 published October 5, 1988 and in U. S. Patent No. 4,571,396. The nalidixic acid analog 6-bromo-1,4-dihydro-5,7-dimethyl-1-ethyl-4-oxonaphthyridine-3-carboxylic acid is referred to on page 26 of the review article by R. Albrecht, Progress in Drug Research, 21, 9-104 (1977). Fluoroquinolone antibacterial agents having an alkyl group in the 5-position and a basic amino substituent in the 7-position have been described in published European Patent Applications 287951 and 319,906 respectively of Ueda, et al. published October 26, 1988, and Matsumoto, et al., published June 14, 1989 but no naphthyridine compounds are disclosed.

## DETAILED DESCRIPTION OF THE INVENTION

The present compounds are antiinfective agents having potent growth inhibitory activity against a wide variety of microorganisms, including Gram positive and Gram negative bacteria which are pathogenic to mammals, including man. Their activity can be demonstrated in vitro by standard microbiological tests which measure the minimum inhibitory concentrations (MIC). Table 4 which follows at the end of the specification contains MIC values for a selection of compounds of the present invention in comparison with ciprofloxacin which is a fluro quinolone anti-bacterial agent presently in wide-spread commercial use for the treatment of infectious diseases. The test compounds are identified in the table by the Procedure Numbers referring to their preparation.

The various compounds of the present invention exhibit superiority over ciprofloxacin in different respects including inter alia antimicrobial spectrum, and particularly Gram positive antibacterial activity, slower excretion rate, tissue distribution, bioavailability following oral administration, cellular penetration, activity against antibiotic resistant organisms, etc. The compounds of Formula I are useful in therapeutics as such or as pharmaceutically acceptable acid addition and base salts wherein the anion or cation, respectively, does not contribute significantly to the toxicity of the salt and which salts are compatible with the standard and conventional pharmaceutically acceptable carriers and other conventional adjuvants and excipients customarily employed in producing pharmaceutical compositions adapted for oral or parenteral administration. The acid addition salts are formed by conventional techniques involving reaction of compounds of Formula I with mineral acids such as, for example, hydrochloric acid, hydrobromic acid, phosphoric aid, and sulfuric acid, and with, organic carboxylic and sulfonic acids such as, for example, acetic acid, citric acid, maleic acid, succinic acid, benzoic acid, tartaric acid, ascorbic acid, methanesulfonic acid, ethanesulfonic acid, 2-hydroxyethanesulfonic acid, p-toluenesulfonic acid, and the like.

Pharmaceutically acceptable base salts are formed by conventional techniques involving reaction of the compounds of Formula I wherein $R^3$ is hydrogen with alkali (Na,K) and alkaline earth (Ca, Ba, Zn, Mn) metal bases, more preferably with alkali metal bases such as, for example, dilute solutions of sodium hydroxide, and potassium carbonate. Also, pharmaceutically acceptable base salts are formed by conventional techniques involving reaction with amines such as, for example, triethylamine, dibenzylamine, triethanolamine, ethanolamine, N,N′-dibenzylethylenediamine, procaine and equivalent amines.

The pharmaceutical compositions of this invention may be prepared by combining a compound of

Formula I with a solid or liquid pharmaceutically acceptable carrier and, optionally, with pharmaceutically acceptable adjuvants and excipients employing standard and conventional techniques. Solid form compositions include powders, tablets, dispersible granules, capsules, cachets and suppositories. A solid carrier can be at least one substance which may also function as a diluent, flavoring agent, solubilizer, lubricant, suspending agent, binder, tablet disintegrating agent, and encapsulating agent. Inert solid carriers include magnesium carbonate, magnesium stearate, talc, sugar, lactose, pectin, dextrin, starch, gelatin, cellulosic materials, low melting wax, cocoa butter, and the like. Liquid form compositions include solutions, suspensions and emulsions. For example, there may be provided solutions of the compounds of this invention dissolved in water and water-propylene glycol and water-polyethylene glycol systems, optionally containing suitable conventional coloring agents, flavoring agents, stabilizers and thickening agents.

The pharmaceutical compositions are prepared by conventional techniques in unit dosage form containing a non-toxic, antimicrobially effective amount of the active component of Formula I. The non-toxic, effective amount is with reference to treatment of infectious diseases of animals by means of topical and systemic routes of administration.

The quantity of active component of Formula I in the pharmaceutical composition and unit dosage form thereof may be varied or adjusted widely depending upon the particular application, the potency of the particular compound, and the desired concentration. Generally, the quantity of active component will range between 0.5% to about 90% by weight of the composition.

In therapeutic use for treating infections in animals, and especially warm-blooded animals, the compounds or pharmaceutical compositions thereof will be administered at a dosages to obtain and maintain an amount of active component in the blood stream or other specifically involved tissue or organ of the animal undergoing treatment which will be effective to inhibit or terminate the infection. Generally, such antibacterially effective amounts will be in the range of about 0.1 to about 15, more preferably about 1.5 to about 10, still more preferably about 3 to about 7 mg/kg of body weight/day. It is to be understood that the dosages may vary depending upon the requirements of the patient, the severity of the bacterial infection being treated, and the particular compound being used. Also, it is to be understood that the initial dosage administered may be increased beyond the above upper level in order to rapidly achieve the desired blood-level or the initial dosage may be smaller than the optimum and the daily dosage may be progressively increased during the course of treatment depending on the particular situation.

The compounds of Formula I are advantageously administered parenterally, i.e. by injection, for example, by intravenous injection or by other parenteral routes of administration. Pharmaceutical compositions for parenteral administration will generally contain a pharmaceutically acceptable amount of the compound according to Formula I as a soluble salt (acid addition salt or base salt) dissolved in a pharmaceutically acceptable liquid carrier such as, for example, water-for-injection and a buffer to provide a suitable buffered isotonic solution, for example, having a pH of about 3.5-6. Suitable buffering agents include, for example, trisodium orthophosphate, sodium bicarbonate, sodium citrate, N-methylglucamine, L-lysine and L-arginine to name but a few representative buffering agents. The compound according to Formula I generally will be dissolved in the carrier in an amount sufficient to provide a pharmaceutically acceptable injectable concentration in the range of about 1 mg/ml to about 400 mg/ml of solution. The resulting liquid pharmaceutical composition will be administered so as to obtain the above-mentioned antibacterially effective amount of dosage in the range of about 0.1 to about 15, more preferably about 1.5 to about 10, still more preferably about 3 to 7 mg/kg of body weight/day.

The compounds of Formula I are prepared from the intermediates of Formula II

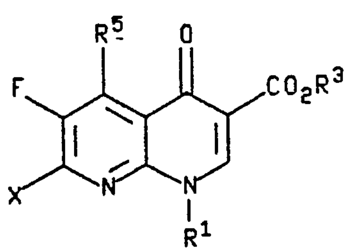

II

in which $R^1$, $R^3$, and $R^5$ have the same meanings as with respect to Formula I, and X is a displaceable leaving group such as a halogen atom, or an organosulfate, or organophosphate ester. The intermediates of Formula II are contacted under displacement reaction conditions with a nitrogen heterocycle, $R^7H$, having one, two, or three heteroatoms selected from oxygen, nitrogen, and sulfur of which at least one is a basic nitrogen atom bearing a displaceable hydrogen atom. The heterocyclic group, $R^7$-, has the same definition

as with respect to Formula I.

The displacement reaction conditions involve contact of the intermediate of Formula II with the heterocycle of the formula $R^7H$ in the presence of at least a chemically equivalent amount relative to said heterocycle of a base (2 equivalents when $R^3$ is H), and in the presence of a reaction inert organic solvent under conditions of time, temperature, and concentration of reactants necessary to effect the desired reaction. Preferred bases are the solvent soluble tertiary amines such as triethylamine, 1-methylpiperidine, diazabicycloundecene, etc. The process has been previously applied in the organic chemical literature for the production of 7-amino substituted 1,4-dihydro-4-oxo-naphthyridine-3-carboxylic acids, and those skilled in the art will be aware of appropriate displacement reaction conditions.

Suitable carboxy-protecting groups, $R^3$, are groups commonly used in organic synthesis to form a readily cleaved ester in those instances where the corresponding acid would undesireably participate in reactions intended for other functional groups in the same molecule. Cleavage of the ester is carried out subsequent to the desired reaction in order to regenerate the carboxyl group. Cleavage is usually by hydrolysis or hydrogenolysis. Many carboxy-blocking groups are known in the literature including methyl, ethyl, benzyl, p-methoxybenzyl, diphenylmethyl, trityl, tert. butyl. Many of the physiologically hydrolyzable ester groups referred to with respect to $R^3$ may also serve as carboxy blocking groups.

The term "physiologically hydrolyzable ester group" used with respect to $R^3$ refers to those esters which are readily metabolized following ingestion by an animal, and in some instances, following parenteral injection, to yield the active acid of Formula I wherein $R^3$ is hydrogen. The metabolic products of the $R^3$ ester group of these esters are harmless in the amounts produced when a compound of Formula I is administered. Some $R^3$ physiologically hydrolyzable ester groups are shown below by formula.

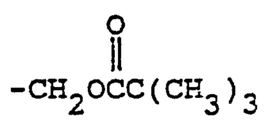

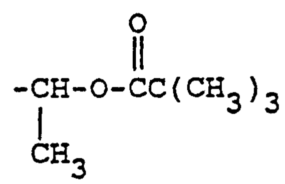

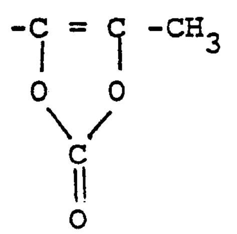

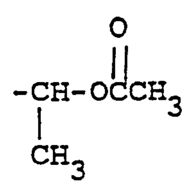

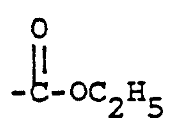

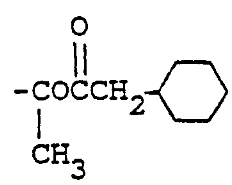

In the following reaction schemes, the symbols $R^1$, $R^5$, and $R^7$ have the same meanings as in Formula I, and $R^8$ is the same as $R^3$ except that $R^8$ is other than hydrogen. In Scheme 1, Step 1, as described below, is regarded as an inventive step. The literature as to methods for the preparation of the tetrasubstituted nicotinic acid derivatives of Formula IV produced by this step is quite limited.

Scheme 1

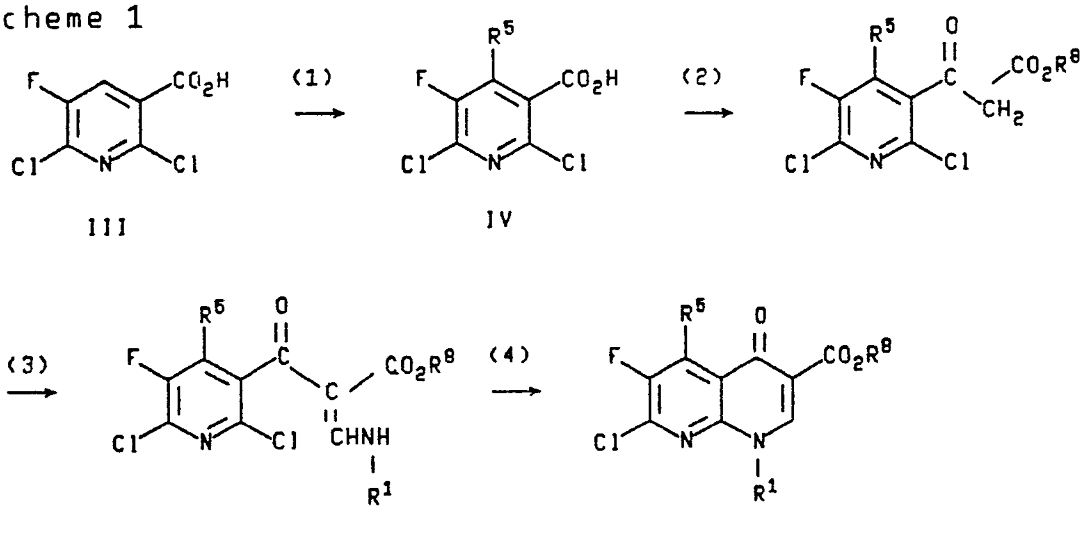


III

IV

Scheme 2

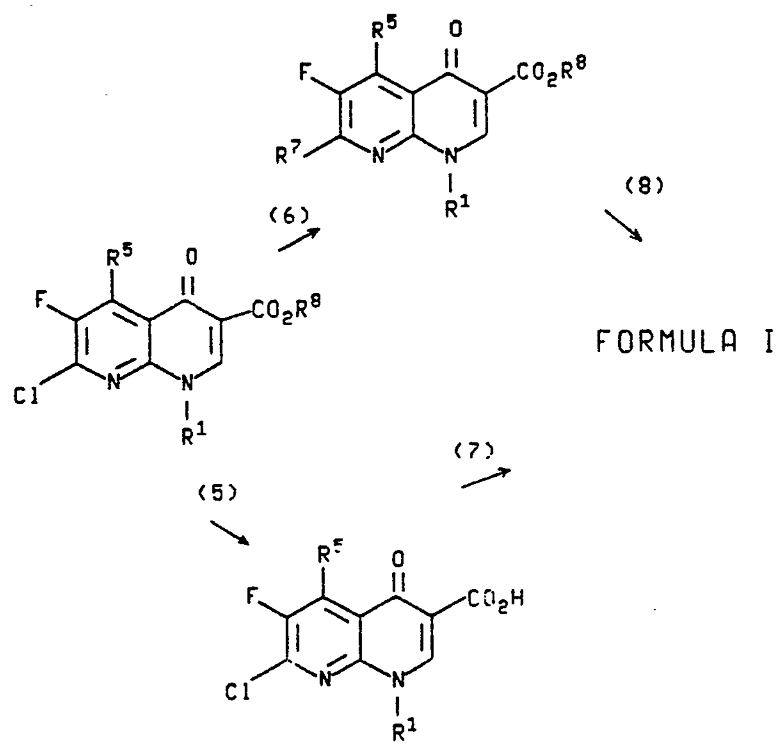


Step (1) is illustrated in Procedures 1, and 15-21 hereof and involves alkylation of the halogenated nicotinic acid of Formula III with an alkyl halide, sulfate, triflate, or phosphate of the formula $R^5X$ wherein $R^5$ and X are as previously defined, preferably being Cl, Br, or I. Low temperature and the presence of a strong base such as a metal alkyl, metal alkoxide, metal hydride in an aprotic solvent facilitate the reaction. Suitable metal alkyls include the alkali metal alkyls such as methyl lithium, and butyl lithium. Metal alkoxides and hydrides are preferably those of the alkali metals such as potassium tert.-butoxide, sodium

hydride, and sodium ethoxide. Aprotic solvents are illustrated by tetrahydrofuran, monoglyme, and diglyme. The reaction is preferably carried out at a temperature of -30° C to -78° C. Equimolecular amounts of the three reactants are employed with an excess of the base and of the reactant $R^5X$ sometimes being desirable.

Step (2) is illustrated in Procedure 2 and involves conversion of the nicotinic acid derivative from Step 1 to the acid chloride which is then used to acylate diethyl malonate via the ethoxymagnesium derivative followed by partial hydrolysis and decarboxylation.

Step (3) is illustrated in Procedures 3, 7-14, and 38. The intermediate produced in Step (2) is converted to the ethoxymethylene derivative using triethylorthoformiate and acetic anhydride, and thence to the iminomethylene compound pictured in the reaction scheme by reaction with the appropriate amine $R^1NH_2$ wherein $R^1$ has the same definition as given for Formula I.

The iminomethylene intermediate produced in Step (3) is cyclized in Step (4) to the corresponding 7-Cl-1,4-dihydro-4-oxo-6-F-1-$R^1$-5-$R^5$-naphthyridine-3-carboxylate shown in the scheme by treatment with sodium hydride or other strong base as is illustrated in Procedures 4, and 39. Heating facilitates this reaction.

Steps (6) and (7) are nitrogen heterocycle displacement reactions for introduction of the $R^7$ substituent as described above. Steps (5) and (8) are esterification or hydrolysis reactions to produce the desired $R^3$ substituent which has been defined with respect to Formula I.

In referring to Formula I to describe the compounds with which the present invention is concerned, it is intended that in those instances where stereoisomers exist due to the presence of an asymmetric carbon atom in one or more of the groups $R^1$, $R^3$, $R^5$, or $R^7$, all stereoisomers and enantiomorphic mixtures thereof are included within the invention. In some instances, such as with respect to the disubstituted-$R^7$ compounds of Procedures 23 and 32, geometric isomers exist. In all such instances, each of the cis- and trans-isomers and mixtures thereof are also included within the invention.

## Description of Specific Embodiments

The following abbreviations are used:

DBU diazabicycloundecene
THF tetrahydrofuran
MeLi methyl lithium
MeOH methanol
DMSO dimethylsulfoxide
PMR proton magnetic resonance
IR infra red
EtOH ethanol
LDA lithiodiisopropylamine
n-BuLi n-butyl lithium
$Et_2O$ ether

The infra red absorption curves were measured on the samples in the solid state diluted with crystalline potassium bromide and compressed into a pellet. The PMR curves were measured on dimethyl-sulfoxide $d_6$ solutions.

## Procedure 1

### 2,6-Dichloro-5-fluoro-4-methylnicotinic Acid

A solution of 10 g of 2,6-dichloro-5-fluoronicotinic acid (0.0476 mole) in 220 mL of anhydrous THF is cooled to -70° C. A solution of MeLi (60 mL, 1.6 m in ether, 0.096 mole) is added in 15 minutes. The reaction mixture is warmed to -30° C and stirred for 2 hours at this temperature. After cooling to -60° C, 8.6 mL of methyl iodide (0.114 mole) are added. The mixture is then warmed to 0° C during 2 hours and treated with water (10 mL). After concentration in vacuo, 500 mL of water are added and the basic aqueous layer is washed with ether (2 x 150 mL). After acidification (pH = 1) with 1N hydrochloric acid, the aqueous layer is extracted with ether (2 x 300 mL). The organic layer is dried over anhydrous magnesium sulfate

and evaporated. The crude product is purified by crystallization from dichloromethane to give 5.69g of pure 2,6-dichloro-5-fluoro-4-methylnicotinic acid, m.p. = 124°C (yield: 53%).

Procedure 2

Ethyl 3-(2,6-Dichloro-3-fluoro-4-methyl-5-pyridyl)-3-oxo-propionate

A mixture of 3 g of 2,6-dichloro-5-fluoro-4-methyl nicotinic acid (13.3 moles) and 2.77 g of $PCl_5$ (13.3 moles) is heated at 100°C for 8 minutes. Then 10 mL of toluene are added and the mixture is concentrated in vacuo. Co-evaporation with toluene is repeated 3 times more to obtain a crude oily acyl chloride.

2.13g of diethylmalonate and 1.36 mL of ethanol are dissolved in 9 mL of anhydrous ether. The resulting solution is added to a suspension of 0.322 g of magnesium in 0.68 mL of ethanol containing a few drops of $CCl_4$. The mixture is refluxed for 2 hours to obtain diethyl ethoxymagnesium malonate. After cooling in an ice bath, the solution of the above acyl chloride in 15 mL of anhydrous ether is added slowly at 5°C. After the addition, the ice bath is removed and the reaction is allowed to stir at room temperature for 2 hours. The reaction mixture is diluted with 20 mL of water, acidified with 0.75 mL of sulfuric acid and extracted with ether. The organic layer is dried over magnesium sulfate and evaporated to dryness. The residue is suspended in 15 mL of water containing 95 mg of p-toluene sulfonic acid. The mixture is refluxed for 2.5 hours. After cooling at room temperature, 45 mL of water is added and the mixture is extracted with ether. The ether fraction is washed with saturated aqueous sodium bicarbonate solution and then water. The organic layer is dried and evaporated to dryness to give the titled compound.

Procedure 3

Ethyl 2-Cyclopropyliminomethylene-3-(2,6-dichloro-3-fluoro-4-methyl-5-pyridyl)-3-oxopropionate

A solution of 2.81 g of the ethyl 3-(2,6-dichloro-3-fluoro-4-methyl-5-pyridyl)-3-oxopropionate (3.55 mole) in triethyl orthoformate (2.4 ml, 14.33 mmoles) and acetic anhydride (2.25 mL, 23.87 mmoles) is heated at 130°C for 3 hours. Additional triethylorthoformate (0.8 mL, 4.78 mmoles) and acetic anhydride (0.45 mL, 4.78 mmoles) are added and the mixture is heated for a further 12 hours. The solution is evaporated under reduced pressure to yield ethyl 2-ethoxymethylene-3-(2,b-dichloro-3-fluoro-4-methyl-5-pyridyl)-3-oxopropionate as an oily residue which is dissolved in 4 mL of ethanol. The solution is cooled in an ice bath and 1.1 mL of cyclopropylamine (15.8 mmoles) are added. The reaction mixture is allowed to stand at room temperature for 3 hours. The solution is evaporated to dryness and the residue is purified by column chromatography on silicagel using ethyl acetate (20%) in hexane for elution to give 2.26 g of the title compound.

Procedure 4

Ethyl 7-Chloro-1-cyclopropyl-1,4-dihydro-6-fluoro-5-methyl-4-oxo-1,8-naphthyridine-3-carboxylate

A 50% sodium hydride in oil suspension (0.327g, 6.82 mmoles) is slowly added to a solution of the product of Procedure 3 (2.24 g, 6.2 mmoles) in dioxane (11 mL). The mixture is heated under nitrogen atmosphere for 3 hours at 80°C. The solvent is evaporated in vacuo and the residue is treated with water (20 mL) and dichloromethane (120 mL). The organic layer is washed with water (2 x 40 mL), dried over magnesium sulfate and evaported to dryness. The residue is recrystallized in ethanol to give 1.22 g of the title compound. m.p. = 176°C (yield: 60%).

Procedure 5

Ethyl 7-[(3S)-3-Amino-1-pyrrolidinyl]-1-cyclopropyl-1,4-dihydro-6-fluoro-5-methyl-4-oxo-1,8-naphthyridine-3-carboxylate

To a suspension of 0.211 g of (3S)-3-aminopyrrolidine dihydrochloride (1.33 mmoles) in acetonitrile (4 mL) there is added 0.63 mL of diazabicycloundecene (DBU), (4.23 mmoles) and 0.34 g of the product of Procedure 4 (1.21 mmoles). The reaction mixture is stirred for 0.5 hours at room temperature. The resulting precipitate is collected by filtration and washed with cold acetonitrile to give 0.912 g of pure product m.p.: 178° C (yield: 43%).

Procedure 6

7-[(3S)-3-Amino-1-pyrrolidinyl]-1-cyclopropyl-1,4-dihydro-6-fluoro-5-methyl-4-oxo-1,8-napthyridine-3-carboxylic Acid Hydrochloride

A suspension of the ethyl ester produced in Procedure 5 (0.332g, 0.88 mmoles) in 1.8 mL of 1N NaOH is refluxed for 15 minutes. After cooling at room temperature and neutralization with 1N HCl, a precipitate is collected by filtration and washed with cold water. The precipitate is suspended in hot ethanol and 1N HCl is added until dissolution. After filtration, the solution is cooled and the resulting precipitate is collected to give 0.186 g of the titled compound, m.p. > 270° C. (yield 54%).

| PMR ($DMSO-d_6$) | S | Relative Area | Multiplicity |
|---|---|---|---|
| | 1.06 | 2 | M |
| | 1.18 | 3 | M |
| | 2.12 | 1 | M |
| | 2.30 | 1 | M |
| | 2.70 | 3 | D (1.8 Hz) |
| | 3.65 | 1 | M |
| | 3.94 | 4 | M |
| | 8.60 | 1 | S |

IR ($cm^{-1}$) 3450, 2880, 1700, 1630, 1505, 1455, 1350, 1240, 1040, 880, 815, 735, 620, 585
Elemental Anal. Found: C, 50.56: H, 5.19; N, 13.82
$[\alpha]_D$ = +15.81° (C=1, 1:1 0.1N HCl/MeOh)

Procedures 7-14

By the substitution of the following primary amines $R^1NH_2$, in equimolar amount for the cyclopropylamine specified in Procedure 3 various ethyl 2-($R^1$-iminomethylene)-3--(2,6-dichloro-3-fluoro-4-methyl-5-pyridyl)-3-oxopropionates are obtained in similar fashion to that described in the procedure. The latter are then transformed in the manner of Procedures 4,5, and 6 to yield various compounds of Formula I.

Table 1
Products of Procedures 7-14
7-((3S)-3-Amino-1-pyrrolidinyl)-1-$R^1$-1,4-dihydro-6-fluoro--5-methyl-1,8-naphthyridine-3-carboxlic Acid Hydrochloride.

| Proc.No. | Amine | $R^1$ |
| --- | --- | --- |
| 7* | 1,1-Dimethylethyl | 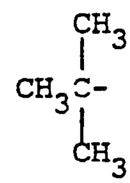 |
| 8 | 2-Fluoroethylamine | $FCH_2CH_2$ - |
| 9* | 4-fluoro aniline | 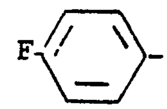 |
| 10* | Ethylamine | $C_2H_5$ - |
| 11* | 1-Methylcyclopropylamine | 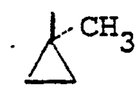 |
| 12 | 3-Trifluoromethylaniline | 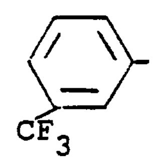 |
| 13 | 1,1-Dimethyl-2-fluoro ethylamine | 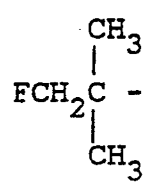 |
| 14 | Cyclopropylmethylamine | 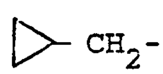 |

*characterizing physical data and procedures are given below following Table 3.

Procedures 15 - 21

By substitution of the following $R^5X$ reactants wherein $R^5$ is as defined for Formula I and X is a displacable inorganic ester leaving group such as halide, sulfate, triflate, or phosphate in chemically equivalent amount for the methyl iodide in Procedure 1, various 2,6-dichloro-5-fluoro-4-$R^5$-nicotinic acids are produced. The latter are then transformed according to the methods of Procedures 2,3,4,5 and 6 in sequence to provide the following compounds of Formula I.

Table 2

Products of Procedures 15 - 21

7-(3(S)-Amino-1-pyrrolidinyl)-1-cyclopropyl-1,4-dihydro-6--fluoro-5-$R^5$-1,8-naphthyridine-3-carboxylic Acid Hydrochloride.

| Proc.No. | $R^5X$ Reactant | R5 |
|---|---|---|
| 15 | Vinyl Triflate | $CH_2$=CH- |
| 16 | n-Butyl Bromide | n-$C_4H_9$ - |
| 17* | Ethyl Iodide | $C_2H_5$- |

* A detailed procedure for the preparation of this compound is given below following Table 3.

Table 2 (cont).

| Proc.No. | Amine | $R^5$ |
|---|---|---|
| 18 | Allyl Bromide | $CH_2$= $CHCH_2$ - |
| 19 | Benzyl Bromide | $C_6H_5CH_2$ - |
| 20 | Cyclopropylmethyl Bromide | 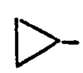 - $CH_2$ - |
| 21 | Cyclohexyl Bromide | 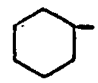 |

Procedures 22-37

Various nitrogen heterocycles are substituted for (3S)-3-aminopyrrolidine in the process of Procedure 5

to provide other compounds of Formula I with various 7-substituents corresponding to the heterocycle used in the modified process. Non-aromatic nitrogen heterocycles having one or two rings, and a nitrogen hetero atom are preferred. Some examples are shown in Table 3.

Table 3

Procedures 22-37

1-Cyclopropyl-1,4-dihydro-6-fluoro-5-methyl-7-$R^7$ naphthyridine-3-carboxylic Acid

| Proc.No. | Heterocycle | $R^7$ |
|---|---|---|
| 22 | Pyrrolidino | 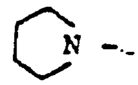 |
| 23 | trans-3-Amino-4-methyl pyrrolidine | 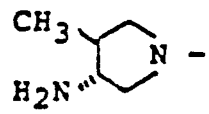 |
| 24 | 1R,4R-2,5-Diazabicyclo-[2.2.1]heptane | 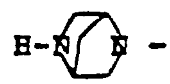 |
| 25 | 8-Methyl-3,8-diazabicyclo [3.2.1]octane | 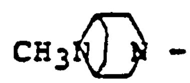 |
| 26 | 3-(aminomethyl)morpholine | 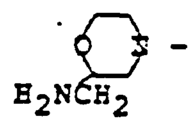 |
| 27 | Piperidine | 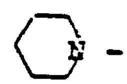 |
| 28 | 3-(aminomethyl)pyrrolidine | 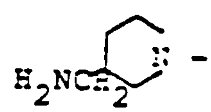 |
| 29 | 3-Ethylaminomethyl-pyrrolidine | 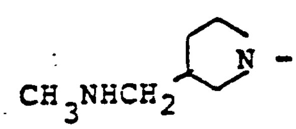 |
| 30* | Piperazine | 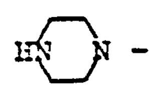 |

Table 3 (continued)

| Proc.No. | Heterocycle | $R^7$ |
|---|---|---|
| 31 | 3-Ethylaminopyrrolidine | 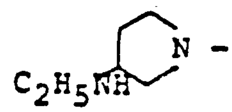 |
| 32 | cis-3-(Aminomethyl)--4- fluoropyrrolidine | 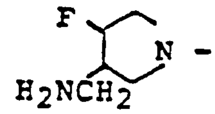 |
| 33 | 1-Methylpiperazine | 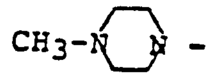 |
| 34* | 3-Methylpiperazine | 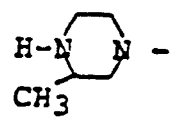 |
| 35 | trans-2,5-Dimethyl piperazine | 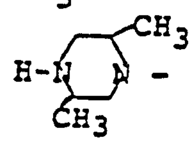 |
| 36 | 2,5-Diaza-2-methyl [2.2.1]bicycloheptane | 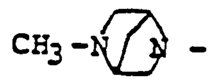 |
| 37 | 3-Aminomethyl thiamorpholine | 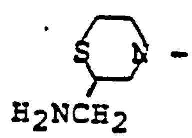 |

*Detailed procedures for the preparation of these compounds are given below following Table 3.

Physical Data for Procedures 7,9,10,11, 17, 30 and 34

Product of Procedure 7

7-[(3S]-3-Amino-1-pyrrolidinyl]-1,4-dihydro-1-(1,1-dimethyl-ethyl)-6-fluoro-5-methyl-1,8-naphthyridine-3-carboxylic Acid Hydrochloride

| PMR (DMSO-$d_6$) | S | Relative Area | Multiplicity |
|---|---|---|---|
| | 1.80 | 9 | S |
| | 2.11 | 1 | M |
| | 2.40 | 1 | M |
| | 2.60 | 3 | D |
| | 3.90 | 5 | M |
| | 8.80 | 1 | S |

IR ($cm^{-1}$) 610, 760, 800, 1180, 1350, 1450, 1500, 1620, 1700, 2430, 3430
Elemental Anal. Found: C, 51.27; H, 6.04; N, 12.91

Procedure 9

7-[(3S)-3-Amino-1-pyrrolidinyl]-1-(4-fluorophenyl)-5-methyl-6-Fluoro-1,4-dihydro-4-oxo-1,8-naphthyridine-3-carboxylic acid

(a.)Ethyl 2(4-Fluorophenyliminomethylene)-3-(2,6-dichloro-4-methyl-3-fluoro-5-pyridyl)-3-oxopropionate

To 4.01 g (0.0114 moles) of 2-ethoxymethylene-3-(2,6-dichloro-3-fluoro-4-methyl-5-pyridil)-3-oxopropionate prepared as described in Procedure 3 dissolved in 1.9 ml of ethanol cooled at o° C there is added over a period of 10 minutes 1.26 g (0.0114 moles) of 4 fluorophenylaniline dissolved in 2 ml of ethanol. After 20 hours at room temperature the solvent is evaporated, and the resulting oil is purified with chromatographic column to yield 2.48 g of the titled compound (yield: 66% (oil).

(b.)Ethyl 7-Chloro-1-(4-fluorophenyl)-1,4-dihydro-5-methyl-4-oxo-1.8-naphthyridine-3-carboxylate

To a solution of 2.46 g (0.00596 moles) of the intermediate producted by the preceding Procedure 9 (a.) in 25 ml of dry $CH_3CN$ are added 802 mg of $K_2CO_3$ (0.00592 moles). After 1 hour of heating at reflux, the solvent is evaporated and the resulting product is dissolved in $CH_2Cl_2$, washed with water and the resulting compound is recrystallized in MeOH to give 1.62 g of the titled compound as a solid mp 230° C (yield 72%).

(c.)Ethyl 7-[(3S]-3-Amino-1-pyrrolidinyl]-1-(4-fluorophenyl)-1,4-dihydro-6-fluoro-5-methyl-4-oxo-1,8-naphthyridine-3-carboxylate

A mixture of 1 g (0.00396 moles) of the intermediate produced in Procedure 9(b.), and 630 mg (0.00396 moles) of 3 (S) aminopyrrolidine dihydrochloride and 1,2 ml of DBU (0.00792 moles) in 10 ml of dry $CH_3CN$, is heated at 90° C for 1 hour. The resulting precipitate is filtered and dried to give 710 mg of the title compound as a solid, m.p. 260° C (yield 63%).

(d.)7-[3-(S)Amino-1-pyrrolidinyl]-1-(4-fluorophenyl)-5-methyl-6-fluoro-1,4-dihydro-4-oxo-1,8-naphthyridine-3-carboxylic acid Hydrochloride

The intermediate produced in the preceding Procedure 9(c.), 710 mg (0.00165 moles), is added to a mixture of 6.6 ml of 1N (0.0066 moles) NaOH and 3 ml EtOH and refluxed 45 minutes, filtered, the solution is adjusted at pH 7.2 with 2N HCl, the precipitate is collected by filtration, washed with water, dried to give 530 mg of the title compound (yield 80%). A suspension of 490 mg (0.00112 moles) of this material in 6 ml of MeOH is refluxed and 2.4 ml of HCl 1N is added and the solution is filtered off to give 370 mg

(mp>260° C) of the title compound.

| PMR | S | Relative Area | Multiplicity |
|---|---|---|---|
| | 2.2 | 2 | M |
| | 2.75 | 3 | D(3.3 HZ) |
| | 3.6 | 4 | M |
| | 3.85 | 1 | M |
| | 7.45 | 2 | T |
| | 7.65 | 2 | M |
| | 8.3 | 2 | M |
| | 8.65 | 1 | S |

IR ($cm^{-1}$) 3406, 3229, 2981, 2547, 1721, 1543, 1346, 1012, 886, 615.
Elemental Anal. Found: C, 50.92; H, 4.53; N, 11.96
$[\alpha]_D$ = +18.8° (C=0.5; 0.1N HCl-MeOH, 4:6)

Product of Procedure 10

7-[(3S)-3-Amino-1-pyrrolidinyl]-1-ethyl-1,4-dihydro-6-fluoro-5-methyl-4-oxo-1.8-naphthyridine-3-carboxylic Acid Hydrochloride Monohydrate

| PMR | S | Relative Area | Multiplicity |
|---|---|---|---|
| | 1.35 | 3 | T |
| | 2.30 | 2 | M |
| | 2.65 | 3 | D |
| | 4.0 | 5 | M |
| | 4.43 | 2 | Q |
| | 8.55 | 2 | S |
| | 8.9 | 1 | S |
| | 15.6 | 1 | S |

IR ($cm^{-1}$) 3474, 2878, 1684, 1546, 1344, 995, 610.
Elemental Anal. Found: C, 49.57; H, 522; N, 14.54.
MOL. WT. 388.16 M.P. > 260° C
$[\alpha]_D$ = +18.4° (C=0.5; 0.1N HCl-MeOH; 6:4).

Product of Procedure 11

7-[3(3S)-3-Amino-1-pyrrolidinyl]-1,4-dihydro-6-fluoro-1-(methyl-1-cyclopropyl)-5-methyl-4-oxo-1,8-naphthyridine-3-carboxylic Acid Hydrochloride

| PMR | S | Relative Area | Multiplicity |
|---|---|---|---|
| | 1.2 | 4 | M |
| | 1.60 | 3 | S |
| | 2.25 | 2 | M |
| | 2.70 | 3 | D |
| | 4 | 5 | M |
| | 9.5 | 2 | M |
| | 9.7 | 1 | S |

IR ($cm^{-1}$) 3433, 2792, 2361, 1692, 1544, 1345, 806,535.
Elemental Anal. Found: C, 53.71; H, 5.58; N, 14.01
MOL. WT. 369.84 M.P.> 260° C
$[\alpha]_D$ = +25.4° (C=1, 0.1N HCl-MeOH; 1:1)

Procedure 17

7-((3S)-3-Amino-1-pyrrolidinyl)-1-cyclopropyl-1,4-dihydro-5-ethyl-6-fluoro-4-oxonaphthyridine-3-carboxylic Acid

(a.) 2,6-Dichloro-4-ethyl-5-fluoro-nicotinic Acid

To a solution of 52 mmol of LDA in 21 mL hexane and 11 mL THF cooled at -75° C was added dropwise a solution of 5g (23.5 mmol) of 2,6-dichloro-5-fluoro-nicotinic acid in 80 mL THF in 45 min. After the addition of the acid the temperature was kept at -75° C for two hours and then 1 hr at -60° C. The reaction mixture was cooled again at -75° C and a solution of 11.6 mL (144 mmol) of ethyl iodide in 15 mL THF was added dropwise. The temperature raised at -20° C in 1 hr and then kept at -20° C for 1 hr. At that time 25 mL of a saturated solution of ammonium chloride was dropped into the solution, the temperature kept below -5° C. The mixture was allowed to stand at room temperature and was made acid with 50 mL 2N HCl. The mixture was extracted with 4x 50 mL of ether dried over MgSO4 and evaporated to give 5.1 g of an oil, crystallized from 100 mL of toluene, 4.46 g of the title compound, purity 80% (NMR).

(b.) 2,6-Dichloro-4-ethyl-5-fluoro-nicotinic Acid Chloride

A mixture of 2.54 g (10.6 mmol) of the above acid (Procedure 17(a.)) and the 2.21 g of $PCl_5$ was heated 8 min. at 110° C in a pre-heated oil bath. The solution was cooled and evaportated at 50° C. The mixture was dissolved in toluene, and the solvent evaporated to give 2.35 g of the title compound as an oil. yield 89%.

(c.) Ethyl 3-(2,6-Dichloro-4-ethyl-3-fluoro-5-pyridinyl)-3-oxo-2-carboxyethylpropanoate

To a suspension fo 0.27 g (11.7 mmol) of magnesium in 0.33 mL of CC14 and 0.63 mL of EtOH stirred 5 min. was added dropwise 1.77 mL (11.7 mmol) of diethyl malonate in 1.23 mL EtOH and 7.9 mL Et20 in order to obtain a gentle reflux. The reflux was maintained for 1h 30 min. The mixture was cooled at 5° C and 2.35 g (9.1 mmol) of the above acid chloride from Procedure 17(b.) in 7.9 ml ET20 was added. The reaction mixture was stirred 10 min. at 5° C and 1h 15 min. at 20° C, poured into a mixture of 3.2 mL conc. H2SO4 and 11 g of ice. After extraction with Et20 it was obtained 3.86 g of the title compound as an oil. yield 100%.

(d.) Ethyl 3-(2,6-Dichloro-4-ethyl-3-fluoro-5-pyridinyl)-3-oxopropanoate

To a suspension of 3.86 g (10.1 mmol) of the above ester (Procedure 17(c.)) in 8 mL H2O was added 23 mg of p-toluenesulfonic acid hydrate. The mixture was refluxed 2 h. The mixture was cooled and diluted with 8 mL H2O and extracted with ether. The organic layer was washed three times with brine, dried ($MgSO_4$) to yield 2.62 g of the title compound as an oil. yield 93%.

(e.) Ethyl 3-(2,6-Dichloro-4-ethyl-3-fluoro-5-pyridinyl)-3-oxo-2-ethoxymethylenepropanoate

A mixture of 2.62 g(8.5 mmol) of the above keto-ester (Procedure 17(d.)) in 2.1 mL (12.7 mmol) of triethylorthoformate and 2.02 mL (21 mmol) of acetic anhydride was heated at 145° C (external) while AcOEt was distilled as fast as it was formed for 1 h. The excess of reactants were distilled at 110° C under vacuum for 1 h to give 2.68 g of the title compound as an oil. yield 87%.

(f.) Ethyl

3-(2,6-Dichloro-4-ethyl-3-fluoro-5-pyridinyl)-3-oxo-2-(((cyclopropyl)amino)methylene)propanoate

To a solution of 2.68 g (7.3 mmol) of the above ester (Procedure 17 (e.)) in 5.7 mL EtOH at 5° C was added 0.52 mL (7.3 mmol) of cyclopropylamine in 10 min. After 1 h at room temperature the solvent was evaporated to give 2.79 g of an oil which was chromatographed over silicagel (Hexane/AcOEt: 80/20) to provide 1.45 g of the title compound as an oil. yield 53.5%.

(g.) Ethyl

7-Chloro-5-ethyl-6-fluoro-1-cyclopropyl-1,4-dihydro-4-oxo-1,8-naphthyridine-3-carboxylate

A mixture of 1.26 g (3.35 mmol) of the above enamine (Procedure 17(f.)) and 0.46 g (3.35 nmol) of $K_2CO_3$ in 15 mL $CH_3CN$ was heated at 80° C for 6 h 30 min. The reaction mixture was coiled and evaporated. The residue was partitioned between $CH_2Cl_2$ and pH 5 buffer. After extraction, and drying (MgSO4) there was obtained 1.18 g of crude product, purified in $Et_2O$ to give 0.73 g of the title compound. yield 66.5%. m.p. 156° C.

Anal. calc. for C16 H116 Cl F N2 O3: C, 56.73; H, 4.76; N, 8.27;
Found: C, 56.46; H, 4.85; N, 7.93.

(h.) Ethyl 7-(3-S-Amino-pyrrolidinyl]-5-ethyl-6-fluoro-1-cyclopropyl-1,4-dihydro-4-oxo-1,8-naphthyridine-3-carboxylate

A mixture of 0.18 g (1.1 mmol) of 3-S-aminopyrrolidine dihydrochloride and 0.35 g (1.03 mmol) of the above naphthyridine (Procedure 17(g.))in 2.34 mL $CH_3CN$ with 0.53 ml (3.5 mmol) of DBU was stirred 1 h at room temperature. After evaporation of the solvent, the residue was extracted with $CH_2Cl_2$ and $H_2O$, dried ($MgSO_4$) to yield 0.36 g of the title compound. mp. 159° C. yield 90 %.

(i.) 7-((3S)-3-amino-1-pyrrolidinyl)-5-ethyl-6-fluoro-1-cyclopropyl-1,4-dihydro-4-oxo-1,8-naphthyridine-3-carboxylic acid, hydrochloride

A suspension of 0.35 g (0.9 mmol) of the above ester (Procedure 17 (h.)) in 0.9 mL of 2N NaOH was heated under reflux for 15 min. (solubilization). The solution was cooled at 40° C and 0.9 mL of 2N HCl was added. After 1 h at room temperature the precipitate was cooled to give 0.29 g of the amino-acid. m.p. 185° C; yield 91%. A suspension of 0.27 g (0.75 mmol) of this material in 2.5 mL EtOH was acidified with 0.375 mL 2N HCl. The mixture was kept at 5° C for 4 h. The precipitate was collected and dried to yield

0.165g of the title compound. m.p. > 250° C. yield 72.5° C.

Anal. calc. for C18 H21 F N4 03, HCl : C, 54.48; H, 5.59; N, 14.12

Found: C, 52.16; H, 5.44; N, 12.72.

## Procedure 30

### 1-Cyclopropyl-1,4-dihydro-6-fluoro-5-methyl-7-(1-piperazinyl)-4-oxo-1,8-naphthyridine-3-carboxilic Acid Hydrochloride

(a.) Piperazine, 150 mg. (2.26 mmoles), and 0.34 mL of diazabicycloundecen (2.27 mmoles) were dissolved in 7 mL of dry acetonitrile. Ethyl 7-chloro-1-cyclopropyl-1,4-dihydro-6-fluoro-5-methyl-4-oxo-1,8-naphthyridine-3-carboxylate 700 mg. (2.15 mmoles) (Procedure 4) was added and the reaction mixture was stirred at room temperature for 2 hours, under nitrogen. The resulting precipitate was collected by filtration and washed with cold acetonitrile to give 0.38 g of the crude product. The crude product was dissolved in 30 mL of water and 5 mL of 2N HCl. The resulting aqueous solution was washed with dichloromethane (30 x 10 mL) and then made alkaline with 2N sodium hydroxide (pH = 11) and extracted with dichloromethane (6 x 10 mL). The organic layer was dried over magnesium sulfate and evaporated to dryness to give 0.252 g of ethyl 7-(1-piperazinyl)-1-cyclopropyl-1,4-dihydro-6-fluoro-5-methyl-4-oxo-1,8-naphthyridine-3-carboxylate, m.p. 185-188° C.

(b.) A suspension of the ethyl ester produced in Procedure 30 (a.) (0.248 g, 0.66 mmoles) in 0.66 mL of 2N NaOH was refluxed for 10 minutes. After cooling at room temperature and neutralization with 2N HCl (pH = 7.3-7.4) a precipitate was collected by filtration and washed with cold water to give 0.220 g of crude 7-(1-piperazinyl)-1-cyclopropyl-1,4-dihydro-6-fluoro-5-methyl-4-oxo-1,8-naphthyridine-3-carboxylic acid. To a suspension of 0.24 g of this acid in 2 mL of hot ethanol was added 2N HCl until dissolution was complete. After cooling, the precipitate was collected to give 0.217 g of a compound which was recrystallized from ethanol - HCl 1N (1/1) to give 0.100 g of the title compound. m.p. > 270° C

| PMR ($DMSO-d_6$) | S | Area | Multiplicity |
|---|---|---|---|
| | 1.14 | 4 | M |
| | 2.75 | 3 | D (3,2 Hz) |
| | 3.25 | 4 | M |
| | 3.72 | 1 | M |
| | 4.02 | 4 | M |
| | 8.64 | 1 | S |

IR ($cm^{-1}$) = 3450, 2926, 2799, 2724, 2611, 2464, 1724, 1622, 1543, 1449, 1367, 1321, 1244, 1186, 1034, 965, 808, 527, 441.

| | | | |
|---|---|---|---|
| Elemental Analysis Calculated: | C = 53.24; | H = 5.27; | N = 14.64 |
| Found: | C = 53.07; | H = 5.20; | N = 14.17 |

## Procedure 34

### 7-(3-Methyl-1-piperazinyl)-1-cyclopropyl-1,4-dihydro-6-fluoro-5-methyl-4-oxo-1,8-naphthyridine-3-carboxylic Acid Hydrochloride

(a.) To a solution of 0.238 g of 2-methylpiperazine (2.38 mmoles) and 0.32 mL of diazabicycloundecene (2.14 mmoles) in 7 mL of dry acetonitrile were added 0.690 g of ethyl 7-chloro-1-cyclopropyl-1,4-dihydro-6-fluoro-5-methyl-4-oxo-1,8-naphthyridine-3-carboxylate (2.12 mmoles) from Procedure 4. The reaction mixture was stirred at room temperature for 3 hours under nitrogen. After cooling in an ice bath, the precipitate was collected by filtration and washed with cold acetonitrile to give 0.394 g of ethyl 7-(3-methyl-1-piperazinyl)-1-cyclopropyl-1,4-dihydro-6-fluoro-5-methyl-4-oxo-1,8-naphthyridine-3-carboxylate, m.p. 177-178°C.

(b.) A suspension of 0.364 g of the ethyl ester produced in Procedure 34 (a.) (0.94 mmoles) in 0.94 mL of 2N NaOH was refluxed for 15 minutes. After cooling and neutralization with 2N HCl (pH=7.2) a precipitate was collected by filtration, washed with cold water and dried in vacuo to give 0.294 g of crude 7-(3-methyl-1-piperazinyl)-1-cyclopropyl-1,4-dihydro-6-fluoro-5-methyl-4-oxo-1,8-naphthyridine-3-carboxylic acid. To a suspension of 0.154 g of this Acid in 3.2 mL of hot ethanol was added 1N HCl until dissolution. After filtration, the solution was cooled and the resulting precipitate was collected to give 0.128 g of the title compound. m.p. > 270°C

| PMR (DMSO-$d_6$) | S | Relative Area | Multiplicity |
|---|---|---|---|
| | 1.20 | 4 | M |
| | 1.32 | 3 | D (5.6 Hz) |
| | 2.74 | 3 | D (3,2 Hz) |
| | 3.45 | 6 | M |
| | 4.45 | 2 | M |
| | 8.63 | 1 | S |
| | 9.42 | 1 | M (exchangeable) |

IR ($cm^{-1}$) 3441, 2938, 2802, 2703, 2668, 2570, 2466, 2361, 1729, 1623, 1506, 1454, 1418, 1325, 1303, 1269, 1106, 966, 809, 436.

| Elemental analysis | | | |
|---|---|---|---|
| Calculated: | C = 54.48; | H = 5.59; | N = 14.12 |
| Found: | C = 54.06; | H = 5.58; | N = 13.85 |

## Procedure 38

### Ethyl 2-(2,4-Difluorophenylaminomethylene)-3-(2,6-dichloro-3-fluoro-4-methyl-5-pyridyl)-3-oxopropanoate

A solution of 2.83g (3.58 mmoles) of ethyl 3-(2,6-dichloro-3-fluoro-4-methyl-5-pyridyl)-3-oxopropanoate (Procedure 2) in triethylorthoformate (0.34 mL, 5.64 mmoles) and acetic anhydride (0.67 mL, 7.05 mmoles) was heated on an oil bath at 140°C for 11 h. The mixture was concentrated in vacuo to a thick residue. The oily residue was dissolved in 3 mL of ethanol. The solution was cooled to +2°C and 0.356g (2.76 mmole) of 2,4-difuoroaniline was added. After being stirred at 2°C, the mixture was warmed at room temperature for 1.5 h. The solution was evaporated to dryness and the residue chromatographied on silica gel (silicagel 60, 70-230 mesh, 120g, eluant: Hexane - Ethyl Acetate - 90/10, 400 mL then Hexane -EthylAcetate 85/15 -1L) to give 0.325 g of the title compound.

## Procedure 39

Ethyl 7-Chloro-1-(2,4-difluorophenyl)-1,4-dihydro-6-fluoro-5-methyl-4-oxo-1.8-naphthyridine-3-carboxylate

A 50% sodium hydride in oil suspension (0.045mg, 1.98 mmoles) was slowly added to a solution of ethyl 2-(2,4-difluorophenylmethylene)-3-(2,6-dichloro-3-fluoro-4-methyl-5-pyridyl)-3-oxopropionate (0.715 g, 1.65 mmoles) in dioxane (2.8 mL). The mixture was heated under nitrogen atmosphere for 45 minutes at 80 °C. The solvent was evaporated and the residue dissolved in dichloromethane. The organic layer was washed with water, dried over magnesium sulfate and evaporated to dryness. The residue was purified by column chromatography (silica gel 60, 70-230 mesh, eluant; $CH_2Cl_2$, MeOH, 98/2) to give 0.356 g of pure title compound (yield: 54%)

Procedure 40

Ethyl 7-[3(S)-Amino-1-pyrrolidinyl]-1-(2,4-difluorophenyl)-1,4-dihydro-6-fluoro-5-methyl-4-oxo-1,8-naphthyridine-3-carboxylate

To a suspension of a 0.155g. (0.97mmole) of 3(S)-aminopyrrolidine dihydrochloride in 3.5 mL of acetonitrile were added 0.46 mL of diazabicycloundecene (DBU) (3.09 mmoles) and 0.35 g of ethyl 7-chloro-1-(2,4-difluorophenyl)-1,4-dihydro-6-fluoro-5-methyl-4-oxo-1,8-naphthyridine-3-carboxylate (0.88 mmole). The mixture was stirred for 45 minutes at room temperature. The solid that formed was collected by filtration and washed with cold acetonitrile to give 0.192 g of the titled compound, m.p. = 210 °C (yield = 49%).

Procedure 41

7-[(3S)-3-Amino-1-pyrrolidinyl]-1-(2,4-difluorophenyl)-1,4-dihydro-6-fluoro-5-methyl-4-oxo-1,8 naphthyridine-3-carboxylic Acid Hydrochloride

A suspension of 0.281 g (0.63 mmole) of ethyl 7-[(3S)-amino-1-pyrrolidinyl]-1-[2,4-difluoropheny!)-1,4-dihydro-6-fluoro-5-methyl-4-oxo-1,8-naphthyridine-3-carboxylate in 1.26 mL of 1N NaOH was refluxed for 0.5 hours. After cooling and neutralization with 1N HCl, a precipitate was collected by filtration and washed with water. This precipitate was suspended in 2 mL of ethanol and 0.5 mL of 1N HCl was added. The mixture was heated until dissolution, filtered while hot, cooled. The solution was evaporated to dryness and the residue triturated with ether to give a solid which was recrystallized in 1 mL of ethanol. 0.132 g of the titled compound was obtained. m.p. > 260 °C.

IR ($cm^{-1}$)3419, 2884, 1721, 1626, 1505, 1448, 1350, 1147, 968, 813, 541. Elemental Anal. Found: C, 48.38; H, 3.86; N, 10.79; ash, 0.67. $[\alpha]_D$ = +15.37 (C = 1, 0.1N HCl/MeOH)

Procedure 42

Ethyl 1-Cyclopropyl-1,4-dihydro-6-fluoro-7-[(3S)-3-hydroxy-1-pyrrolidinyl]-5-methyl-4-oxo-1,8-naphthyridine-3-carboxylate

To a suspension of 0.260 g (2.10 mmoles of (3S)-3-Hydroxy pyrrolidine hydrochloride in 4,5 mL of dry acetonitrile there was added 0.55 mL (3.67 mmoles) of DBU and 0.460 g (1.41 mmoles) of ethyl 7-chloro-1-cyclopropyl-1,4-dihydro-6-fluoro-5-methyl-4-oxo-1,8-naphthyridine-3-carboxylate prepared as described in Procedure 4. The reaction mixture was stirred at 60 °C for 0.5 hour. After cooling the solvent was removed *in vacuo*. The solid residue was washed with water and recrystallized from acetonitrile to give 0.331 g of the title compound.

m.p. = 216-217 °C

$[\alpha]_D$ = + 21.76° (C=1,$CHCl_3$)

| Elemental analysis: $C_{19}H_{22}FN_3O_4$ | Calculated | Found |
|---|---|---|
| C | 60.79 | 60.84 |
| H | 5.91 | 6.00 |
| N | 11.19 | 11.16 |

## Procedure 43

1 Cyclopropyl-1,4-dihydro-6-fluoro-7-[(3S)-3-hydroxy-1-pyrrolidinyl]-5-methyl-4-oxo-1,8-naphthyridine-3-carboxylic acid

A suspension of the ethyl ester produced in Procedure 42 (0.300 g, 0.80 mmoles) in 0.8 mL of 2N NaOH was refluxed for 2 hours. The mixture was cooled and acidified to pH 1 with 1N HCl. The precipitate was collected and recrystallized from a methanol-dichloromethane mixture to give 0.132 g of the title compound, m.p. > 270° C.

| Elemental analysis: $C_{17}H_{18}FN_3O_4$ | Calculcated | Found |
|---|---|---|
| C | 58.78 | 58.92 |
| H | 5.22 | 5.35 |
| N | 12.1 | 12.11 |

| PMR (DMSO-$d_6$) | S | Area | Multiplicity |
|---|---|---|---|
| | 1.04 | 2 | D |
| | 1.16 | 2 | D |
| | 1.98 | 2 | M |
| | 2.67 | 3 | D (3.3 Hz) |
| | 3.70 | 4 | M |
| | 4.41 | 1 | M |
| | 5.06 | 1 | D (-OH) |
| | 8.53 | 1 | S |

IR ($cm^{-1}$) = 3428, 1718, 1626, 1550, 1503, 1413, 1324, 1227, 1207, 1154, 1094, 1033, 1005, 876, 845, 809, 726, 612, 577, 534, 478, 431.

$[\alpha]_D$ = +21.2° (C = 0.5 NaOH O,1N)

## PROCEDURE 44

Ethyl 7-(1-Piperazinyl)-1-cyclopropyl-1,4-dihydro-5-ethyl-6-fluoro-4-oxo-1,8-napthyridine-3-carboxylate

A mixture of 0.28 g (3.2 mmol) of piperazine and 0.35 g (1.03 mmol) of the naphthyridine produced in Procedure 17(g.)in 5.5 mL CH3CN was refluxed for 30 mn. After evaporation of the solvent, the residue was taken up in 5 mL $H_2O$ ice-cooled to yield 0.38 g of the title compound. m.p. 165° C. Yield 95%.

PROCEDURE 45

7-(1-Piperazinyl)-1-cyclopropyl-1,4-dihydro-5-ethyl-6-fluoro-4-oxo-1,8-naphthyridine-3-carboxylic acid hydrochloride

A suspension of 0.36 g (0.92 mmol) of the ester produced in Procedure 44 in 0.92 mL of 2N NaOH was heated under reflux for 15 mn (dissolution). The solution was cooled and made acid with 0.92 mL 2N HCl. The precipitate was stirred at 5° C for 30 mn and filtered to give 0.33 g of the carboxylic acid. m.p. 235° C.

A suspension of 0.31 g (0.86 mmol) of the carboxylic acid in 2.5 mL of EtOH was warmed and acidified with 0.43 mL 2N HCl. After 5 mn of solubilisation a precipitate was formed which was collected, dried to give 0.3 g of the title compound. m.p.>25C° C. Yield 91%.

| Anal. calc. for C18 H21 F N4 03,HCl: | | | |
|---|---|---|---|
| | C, 54.48; | H, 5.59; | N, 14.12. |
| Found: | C, 52.25; | H, 5.82; | N, 11.88. |

PROCEDURE 46

Ethyl 1-(2,4-Diflurophenyl)-1,4-dihydro- 7-[(3S-3-hydroxy-1-pyrrolidinyl]-6-fluoro-5-methyl-4-oxo-1,8-naphthyridine-3-carboxylate

To a suspension of 0.302 g (2.44 mmoles) of (3S)-3-hydroxy-pyrrolidine hydrochloride in 6.5 mL of dry acetonitrile were added 0.64 mL of DBU (4.26 mmoles) and 0.65 g (1.63 mmoles) of ethyl 7-chloro-1-(2,4 -difluorophenyl)-1,4-dihydro-6-fluoro-5-methyl-4-oxo-1,8 -naphthyridine-3- carboxylate from Procedure 4. The reaction mixture was stirred at room temperature for 0.5 hour. The resulting precipitate was collected by filtration and washed with cold acetonitrile to give 0.648 g of a crude product. This crude product was washed with water and recristallized from acetonitrile to give 0.548 g of the title compound. m.p. = 241° C, $[\alpha]_D$ = 10.52° (C=1, MeOH)

PROCEDURE 47

1-(2 4-Difluorophenyl)- 1,4-dihydro-7-[(3S)-3-hydroxy-1-pyrrolidinyl]-6-fluoro-5-methyl-4-oxo-1,8-naphthyridine-3-carboxylic Acid

A suspension of 0.465 g (1 mmole) of the ethyl ester produced in Procedure 46 in 2 mL of 2N NaOH was refluxed for 1 hour. Then 10 mL of ethanol were added and the resulting solution was refluxed for an additional 0.25 hour. After cooling at room temperature 2 mL of 2N HCl were added and the reaction mixture was concentrated in vacuo. The resulting precipitate was collected by filtration, washed with water and dried to give 0.398 g of a crude product. The crude product was dissolved in 18, 5 mL of chloroform. The organic layer was filtered and evaporated in vacuo to give 0.366 g of the title compound.

m.p. = 268° C.
$[\alpha]_D$ = +4.96° (C=1, $CHCl_3$)

| PMR (DMSO-d6) | S | Area | Multiplicity |
|---|---|---|---|
| | 1.82 | 2 | M |
| | 2.71 | 3 | D (3.2 Hz) |
| | 3.50 | 4 | M |
| | 4.27 | 1 | M |
| | 4.96 | 1 | D (- OH) |
| | 7.32 | 1 | M |
| | 7.54 | 1 | M |
| | 7.75 | 1 | M |
| | 8.72 | 1 | S |

I-R ($cm^{-1}$): 3393, 3080, 2975, 1708, 1625, 1804, 1450, 1440, 1345,1221, 1146, 1098, 969, 810, 530.
Elemental Analysis Found: C = 53.44 - H: 3.65 - N: 9.15
$[\alpha]_D$ = +4.96° (C = 1, $CHCl_3$)

Preparation of 1-(2,4-Difluorophenyl)-1,4-dihydro-7-(3-ethylaminomethyl-1-pyrrolidinyl)-6-fluoro-5-methyl-4-oxo-1,8-naphthyridine-3-carboxylic Acid Hydrochloride

PROCEDURE 48

Ethyl 1-(2,4-Difluorophenyl)-1,4-dihydro-7-(3-ethylaminomethyl-1- pyrolidinyl)-6-fluoro-5-methyl-4-oxo-1,8-naphthyridine-3-carboxylate

To a solution of 0.364 g (2.83 mmoles) of 3-ethylaminomethyl-1-pyrrolidine in 10 mL of acetonitrile were added 0.4 mL (2.67 mmoles) of DBU and 1 g (2.52 mmoles) of ethyl-7-chloro-1-(2,4-difluorophenyl)-1,4-dihydro-6-fluoro-5-methyl-4-oxo-1,8-naphthyridine-3- carboxylate (Procedure 39). The reaction mixture was stirred at room temperature for 1.5 hour. The solvent was removed in vacuo and the residue was dissolved in 25 mL of dichloromethane. The resulting solution was washed with water (4x20 mL), dried over $MgSO_4$ and evaporated to dryness. The solid residue was suspended in 20 mL of water. After acidification to pH = 1 with 2N HCl, the aqueous layer was filtered and adjusted to pH 8.5 with 2N NaOH. The resulting precipitate was collected by filtration to give 0.77 g of the title compound. m.p. = 85° C.

PROCEDURE 49

1-( 2,4-Difluorophenyl)-1,4-dihydro-7-(3-ethylaminomethyl-1-pyrrolidinyl)-6-fluoro-5-methyl-4-oxo-1,8-naphthyridine-3-carboxilic acid.

The ethyl ester prepared in Procedure 48, 0.608 g (1.2 mmoles), was dissolved in a mixture of 2.4 mL of ethanol and 2.4 mL of 1N NaOH. The solution was refluxed for 0.75 hour. After cooling the reaction mixture was concentrated and 10 mL of water was added.

The solution was neutralized with 1N HCl. The precipitate was collected by filtration and dried in vacuo to give 0.425 g of the crude product. This product, 0.368 g, was suspended in 2 mL of acetone and 0.15 mL of concentrated hydrochloric acid was added. The mixture was heated until dissolution. After cooling, a precipitate was collected and washed with cold acetone. The filtrate was evaporated to dryness and the residue recrystallized from 2 mL of ethanol to give 0.083 g. of the hydrochloride salt of the title compound.

m.p. = 226°C.

| PMR (DMSO-d6) | S | Area | Multiplicity |
|---|---|---|---|
| | 1.21 | 3 | T (J = 7 Hz) |
| | 1.73 | 1 | M |
| | 2.07 | 1 | M |
| | 2.5 | 2 | M |
| | 2.73 | 3 | D |
| | 2.92 | 3 | M |
| | 3.33 | 2 | M |
| | 7.32 | 1 | M |
| | 7.54 | 1 | M |
| | 7.76 | 1 | M |
| | 8.9 | 1 | S |

IR (cm-1) = 3427, 2949, 2780, 2744, 1717, 1505, 1441, 1342, 1273, 1147, 968, 809, 530.
Elemental Analysis Found: C: 54,2% - H: 4,9% - N: 10.72%

PROCEDURE 50

7-Chloro-1-cyclopropyl-1,4-dihydro-6-fluoro-5-methyl-4-oxo-1,8-naphthyridine-3-carboxylic Acid.

A suspension of 1.25 g of ethyl 7-chloro-1-cyclopropyl-1,4-dihydro-6-fluoro-5-methyl-4-oxo-1,8-naphthyridine-3-carboxylate (3.85 mmoles) from Procedure 4 in 25 mL of ethanol and 2.5 mL of 1N HCl was refluxed for 1 hour, and then kept overnight at room temperature. A precipitate formed and was collected by filtration, washed with cold ethanol, to afford 0.980 g (86%) of the titled compound, m.p. 215°C.

PROCEDURE 51

7-[(2S)-2-Methyl-4-acetyl-1-piperazinyl]-1-cyclopropyl-1,4-dihydro-6-fluoro-5-methyl-4-oxo-1,8-naphthyridine-3-carboxylic Acid.

Under nitrogen, 0.800 g of 7-chloro-1-cyclopropyl-1,4 -dihydro-6-fluoro-5-methyl-4-oxo-1,8-naphthyridine-3-carboxylic acid (2.69 mmoles) from Procedure 50, and 1.42 g of 1-acetyl-3(S)-3-methyl-piperazine (10.0 mmoles) were heated at 120°C for 5 mm. The precipitate which formed collected by filtration triturated with 10 mL of HCl 0.1N, filtered and washed with cold water to give 0.960 g (89%) of the title compound, m.p. 246°C, $[\alpha]_D$ = +104° (C = 1, $CHCl_3$).

PROCEDURE 52

7-[(2S)-2-Methyl-1-piperazinyl]-1-cyclopropyl-1,4-dihydro-6-fluoro-5-methyl-4-oxo-1,8-naphthyridine-3-carboxylic Acid.

A suspension of 0.900 g of 7-[(2S)-2-methyl-4-acetyl-1-piperazinyl]-1-cyclopropyl-1,4-dihydro-6-fluoro-5-methyl-4-oxo-1,8-naphthyridine-3-carboxylic acid (2.23 mmoles) prepared in Procedure 51 in 5 mL of 2N NaOH was refluxed for 3.5h under nitrogen, then diluted with 10 mL of $H_2O$. After cooling and neutralizing

with 2N HCl, a precipitate formed and was collected and washed with cold water to yield 0.800 g (100%) of the title compound, m.p. 206° C. A portion of this material, 0.700 g, was suspended in 10 mL of 1N HCl and heated until dissolution took place and then kept at room temperature until precipitation of the hydrochloride salt of the title compound was complete; yield 0.670 g (87%), m.p. > 260° C, $[\alpha]_D$ = +133.9° (C = 1, 0.1N HCl).

| PMR (DMSO-d6) | S | Area | Multiplicity |
|---|---|---|---|
| | 1.00 | 2 | M |
| | 1.15 | 2 | M |
| | 1.42 | 3 | D (6.8 Hz) |
| | 2.63 | 3 | D (3.1 Hz) |
| | 3.40 | 6 | M |
| | 4.45 | 1 | M |
| | 4.86 | 1 | M |
| | 8.55 | 1 | S |

IR (cm-1) : 3428, 3013, 2940, 2862, 2775, 2740, 2671, 2503, 2423, 2363, 2255, 1725, 1624, 1542, 1500, 1445, 1380, 1320, 1227, 1174, 1116, 1063, 1034, 994, 952, 863, 809, 765, 734, 671, 616, 583.

PROCEDURE 53

2,6-Dichloro-4-phenyl-5-fluoro-nicotinic Acid.

A solution of 9 mL (22 mmL) of n-BuLi 2.5M in hexane was added to 25 mL dry THF, cooled to -25° C, and treated in dropwise fashion with 3.6 mL (20 mmL) of diisopropylamine in 6 mL THF. The temperature of the solution was then allowed to rise to +5° C in 1 hour. At that time, the solution was cooled at -75° C and a solution of 2.11g (10 mmL) of 2,6 -dichloro-5-fluoronicotinic acid in 25 mL THF was added dropwise during 65 min. The mixture was kept at -75° C for 3 hours 20 minutes. The temperature was then allowed to rise to -60° C for 20 minutes and cooled again at -75° C. Solution of 1M zinc chloride in ether 23 mL (23 mmol) was then added during 6 minutes. The solution was allowed to warm to room temperature during one 1 hour and kept 15 minutes at this temperature. This solution was transferred into a dropping funnel having a rubber septum inlet using a double-ended needle with a slight vacuum and then added dropwise from the funnel to a mixture of 2.45 g (12 mmol) of iodobenzene and 1.01 g (0.88 mmol) of $Pd(PPh_3)_4$ in 12 mL dry THF. The dark red solution was stirred overnight and poured into a saturated solution of $NH_4Cl$ and acidified with 35 mL 2N hydrochloric acid. The aqueous layer was separated and extracted twice with 100 mL $Et_2O$. The organic layers were combined and extracted twice with 25 mL of 10% $NaHCO_3$ solution. The aqueous layers were combined and washed with ether. The combined aqueous solution was cooled in an ice bath and acidified with 4.8 mL conc. HCl and then extracted with 60 mL $Et_2O$. The organic layer was washed with water and dried (MgSO4) to give 2.22 g of crude product. The crude product was stirred in 4 mL toluene to separate the insoluble starting material (0.71 g) filtered, and the filtrate was evaporated to yield 1.2 g of crude product which was chromatographed over silica gel 40-63 µm using $CH_2Cl_2$-MeOH (97:3) as eluant. There was obtained 0.33 g of the title compound, yield 11%, m.p. 159-60° C.

Anal. Code for $C_{12}H_6Cl_2FNO_2$; C:50.38; H:2.11; N:4.90 found C:49.98; H:2.28; N:4.45

$^1H$ NMR δ (ppm) in DMSO-$d_6$: 7.51 (singlet, aromatic H).

$^{19}F$ NMR δ (ppm) in DMSO-d6: -116.8.

IR($cm^{-1}$) 2919, 1718, 1365, 1289.

The product of Procedure 53 can be converted to 5 -phenyl-1,4-dihydronaphthyrid-4-one products of the present invention according to synthetic procedures given herein.

Table 4

| Minimum Inhibitory Concentrations ( in vitro broth dilution method) | | | | | | | |
|---|---|---|---|---|---|---|---|
| | Test Compound | | | | | | |
| Organism | Proc. A.No. | Proc. 41 | Proc. 6 | ciprofloxacin | Proc. 9 | Proc. 30 | 7 |
| S.pneumoniae/Todd Hewitt | A9585 | .03 | .03 | .5 | .03 | .25 | .25 |
| S.pyogenes/Todd Hewitt | A9604 | .03 | .03 | .5 | .03 | .25 | .13 |
| S.agalactiae/Todd Hewitt | A22567 | .06 | .06 | .5 | .13 | .25 | 2.0 |
| E. faccalis | A9809 | .13 | .13 | .5 | .13 | .5 | 0.5 |
| E. faecium | A24885 | 2 | 1 | 4 | 1 | 1 | 16.0 |
| S. aureus/Pen.- | A9537 | .002 | .001 | .13 | .002 | .008 | .0005 |
| S. aureus/50% human serum | A9537S | .002 | .002 | .13 | .004 | .016 | .06 |
| S. aureus/NCCLS strain | A24227 | .001 | .001 | .13 | .002 | .03 | .004 |
| S. aureus/Pen. + | A9606 | .008 | .004 | .25 | .016 | .13 | 016 |
| S. aureus/MR 35 deg.C 24hr | A20699 | .008 | .004 | .25 | .03 | .06 | - |
| S. epidermidis/MR 35 deg. C 24h | A25783 | .008 | .008 | .25 | .06 | .13 | - |
| E. coli/NCCLS strain | A20697 | .0005 | .0005 | .008 | .002 | .002 | .00025 |
| E. coli | A15119 | .0005 | .0005 | .002 | .002 | .002 | .002 |
| K. oxytoca | A20345 | .001 | .001 | .03 | .004 | .016 | .13 |
| K. pneumoniae | A9664 | .008 | .001 | .008 | .002 | .002 | .016 |
| E. aerogenes | A20985 | .002 | .001 | .008 | .004 | .008 | .03 |
| E. cloacae | A9656 | .002 | .001 | .008 | .002 | .004 | .06 |
| Ps. aeruginosa | A9843 | .5 | .25 | .25 | .5 | .5 | 1.0 |

**Claims**

1. A compound having the formula

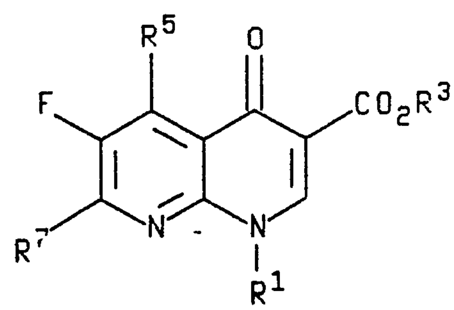

(I)

wherein
$R^1$ is selected from cycloalkyl and cycloalkylalkyl having from 3 to 7 carbon atoms including 3 to 6 ring members and up to 3 substituents selected from methyl and fluorine, lower alkyl having 2 to 6 carbon atoms, lower fluoroalkyl having 2 to 6 carbon atoms and 1 to 3 fluorine atoms, and phenyl or substituted phenyl having 1 or 2 substituents selected from fluorine and trifluoromethyl;
$R^3$ is hydrogen, lower alkyl having 1 to 4 carbon atoms, a physiologically hydrolyzable ester group, or a carboxy-protecting group of the readily removable type conventionally used in organic synthesis;
$R^5$ is selected from lower alkyl having 1 to 6 carbon atoms, lower alkenyl having 2 to 6 carbon atoms, cycloalkyl having 3 to 6 carbon atoms, cycloalkylalkyl having 4 to 7 carbon atoms, aryl having 6 to 10 carbon atoms, and aralkyl having 7 to 11 carbon atoms;
$R^7$ is a nitrogen heterocycle having one or two rings and 4 to 9 ring members and up to two additional heteroatoms selected from O, S, and N, said heterocycle being covalently attached through a ring-N atom

thereof which heterocycle may be unsubstituted or substituted by 1 or 2 substituents having up to 6 carbon atoms and selected from alkyl, alkanoyl, hydroxyalkyl, hydroxy, chlorine, fluorine, amino, alkylamino, dialkylamino, aminoalkyl, alkylaminoalkyl, dialkylaminoalkyl, carboxy, carboxyalkyl, and carboxamido, and the pharmaceutically acceptable acid addition salts thereof, and the pharmaceutically acceptable metal and amine salts of those compounds wherein $R^3$ is H.

2. The compounds of Claim 1 wherein $R^3$ is H.

3. The compounds of Claim 1 wherein $R^1$ is cyclopropyl, 2-fluoroethyl, 2-fluorophenyl, 4-fluorophenyl, 2,4-difluorophenyl, or tert.-butyl.

4. The compounds of Claim 1 wherein $R^5$ is methyl.

5. The compounds of Claim 1 wherein $R^7$ is 1-pyrrolidinyl or substituted 1-pyrrolidinyl.

6. The compounds of Claim 1 wherein $R^7$ is 3-amino-1-pyrrolidinyl, 3(S)-amino-1-pyrrolidinyl, 1-piperazinyl or
4-methyl-1-piperazinyl.

7. The compound of Claim 1 wherein $R^1$ is cyclopropyl, $R^3$ is hydrogen, $R^5$ is methyl, and $R^7$ is 3-amino-1-pyrrolidinyl.

8. The hydrochloride salt of the compounds of Claim 1.

9. The compounds of Claim 1 selected from
7-(3S)-3-amino-1-pyrrolidinyl)-1-cyclopropyl-1,4-dihydro-6-fluoro-5-methyl-4-oxo-1,8-naphthyridine-3-carboxylic acid, and the hydrochloride salt thereof;
7-((3S)-3-amino-1-pyrrolidinyl)-1,4,-dihydro-1-(2,4-difluorophenyl)-6-fluoro-5-methyl-4-oxo-1,8-napthyridine-3-carboxylic acid,
7-((3S)-3-amino-1-pyrrolidinyl)-1,4-dihydro-1-(1,1-dimethylethyl)-6-fluoro-5-methyl-4-oxo-1,8-naphthyridine3-carboxylic acid.
1-cyclopropyl-1,4-dihydro-6-fluoro-5-methyl-7-(1-piperazinyl)naphthyridine-3-carboxylic acid;
1-cyclopropyl-1, 4-dihydro-6-fluoro-5-methyl-7-(4-methyl-1-piperazinyl)napththyridine-3-carboxylic acid;
7-(3-methyl-1-piperazinyl)-1-cyclopropyl-1,4-dihydro-6-fluoro-5-methyl-4-oxo-1,8-naphthyridine-3-carboxylic acid and the hydrochloride salt thereof;
1-cyclopropyl-1,4-dihydro-6-fluoro-7-[(3S)-3-hydroxy-1-pyrrolidinyl]-5-methyl-4-oxo-1,8-naphthyridine-3-carboxylic acid ;
ethyl-7-(1-piperazinyl)-1-cyclopropyl-1,4-dihydro-5-ethyl-6-fluoro-4-oxo-1,8-naphthyridine-3-carboxylate;
7-((3S)-3-amino-1-pyrrolidinyl)-1-cyclopropyl-1,4-dihydro-5-ethyl-6-fluoro-4-oxonaphthyridine-3-carboxylic acid;
7-[(3S)-3-amino-1-pyrrolidinyl]-1-ethyl-1,4-dihydro-6-fluoro-5-methyl-4-oxo-1,8-napthyridine-3-carboxylic acid;
7-[(3S)-3-amino-1-pyrrolidinyl]-1-(4-fluorophenyl)-5-methyl-6-fluoro-1,4-dihydro-4-oxo-1,8-naphthyridine-3-carboxylic acid;
1-(2,4-difluorophenyl)-1,4-dihydro-7-[(4S)-3-hydroxy-1-pyrrolidinyl]-6-fluoro-5-methyl-4-oxo-1,8-naphthyridine-3 carboxylic acid ;
1-(2,4-difluorophenyl)-1,4-dihydro-7-(3-ethylaminomethyl-1-pyrrolidinyl)-6-fluoro-5-methyl-4-oxo-1,8-naphthyridine-3-carboxylic acid ;
7-[3(3S)-3-amino-1-pyrrolidinyl]-1,4-dihydro-6-fluoro-1-(1-methyl-1-cyclopropyl)-5-methyl-4-oxo-1,8-naphthyridine 3 carboxylic acid hydrochloride;
7-[(2S)-2-methyl-1-piperazinyl]-1-cyclopropyl-1,4-dihydro-6-fluoro-5-methyl-4-oxo-1,8-naphthyridine-3-carboxylic acid; and
7-[(2S)-2-methyl-4-acetyl-1-piperazinyl]-1-cyclopropyl-1,4-dihydro-6-fluoro-5-methyl-4-oxo-1,8a-naphthyridine-3-carboxylic acid and the hydrochloride salt thereof.

10. The lower alkyl esters of the compounds of Claim 1.

11. The process for the preparation of a compound of Claim 1 which comprises

i) contacting under reaction conditions, a solution of a compound of Formula III

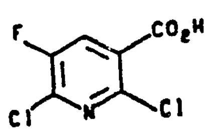

III

in an aprotic solvent and at a temperature in the range of -30° C to -78° C with a strong base and a

compound of the formula $R^5X$ wherein $R^5$ has the same meaning as with respect to Formula IV and is selected from lower alkyl having 1 to 6 carbon atoms, lower alkenyl having 2 to 6 carbon atoms, cycloalkyl having 3 to 6 carbon atoms, cycloalkylalkyl having 4 to 7 carbon atoms, aryl having 6 to 10 carbon atoms, and aralkyl having 7 to 11 carbon carbon atoms and X is a displaceable leaving group for a time period sufficient for production of a substantial amount of the compound of Formula IV

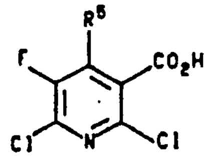

IV

ii)

(a) activating a compound of Formula IV with a halogenating agent,then acylating with dialkyl malonate followed by partial hydrolysis and decarboxylation to produce a compound of Formula

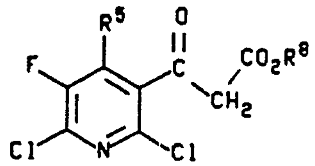

wherein $R^5$ is as previously defined and $R^8$ is the same as $R^3$ except that $R^8$ is other than hydrogen

(b) reacting a compound of step (a) with trialkylorthoformate and then with an appropriate amine $R^1NH_2$ wherein $R^1$ has the same definition as given in Formula I to produce a compound of Formula

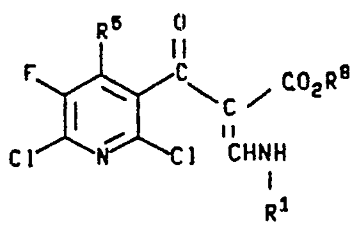

(c) cyclizing a compound of step (b) in the presence of a base to produce a compound of Formula II;

iii) contacting a compound of Formula II

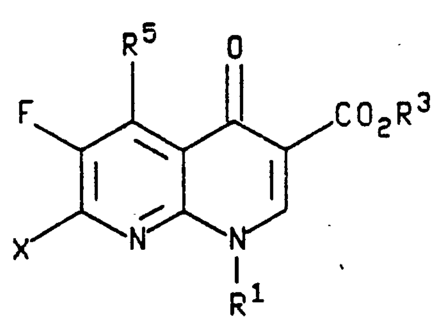

II

wherein $R^1$, $R^3$, and $R^5$ have the same meaning as in Claim 1 and X is a displaceable leaving group selected from halogen, organosulfate, and organophosphate with a heterocycle of the formula $R^7H$ wherein $R^7$ has the same meaning as in Claim 1 under displacement reaction conditions in an aprotic organic solvent in the presence of a solvent soluble tertiary amine base.

12. A pharmaceutical composition, preferably in dosage unit form, containing a non-toxic effective antiinfective amount of at least one compound of Claims 1 to 10 and a pharmaceutical carrier thereof.

13. A process for preparing the pharmaceutical composition of claim 12 which comprises incorporating a non-toxic effective antiinfective amount of at least one compound of claims 1 to 10 into a pharmaceutical carrier.

14. The use of at least one compound of claims 1 to 10 for preparing a pharmaceutical composition for the treatment of an infection in an animal including man.

15. The process for the preparation of a compound of Formula IV

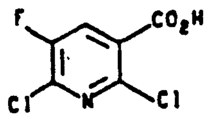

III

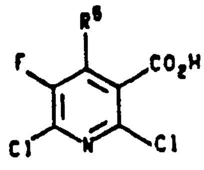

IV

which comprises contacting under reaction conditions, a solution of a compound of Formula III in an aprotic solvent and at a temperature in the range of -30° C to -78° C with a strong base and a compound of the formula $R^5X$ wherein $R^5$ has the same meaning as with respect to Formula IV and is selected from lower alkyl having 1 to 6 carbon atoms, lower alkenyl having 2 to 6 carbon atoms, cycloalkyl having 3 to 6 carbon atoms, cycloalkylalkyl having 4 to 7 carbon atoms, aryl having 6 to 10 carbon atoms, and aralkyl having 7 to 11 carbon atoms and X is a displaceable leaving group for a time period sufficient for production of a substantial amount of the compound of Formula IV.

16. The process for the preparation of a compound of Formula II

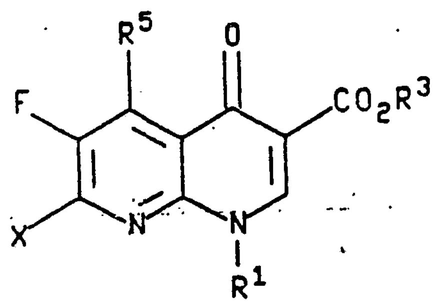

II

in which $R^1$, $R^3$, and $R^5$ have the same meanings as with respect to Formula I, and X is a displaceable leaving group which comprises

(a) activating a compound of Formula IV with a halogenating agent then acylating with dialkyl malonate followed by partial hydrolysis and decarboxylation to produce a compound of Formula

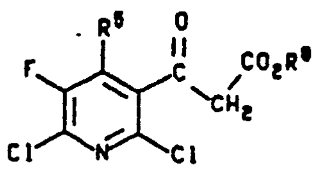

wherein $R^5$ is as previously defined and $R^8$ is the same as $R^3$ except that $R^8$ is other than hydrogen,

(b) reacting a compound of step (a) with trialkylorthoformate and then with an appropriate amine $R^1NH_2$ wherein $R^1$ has the same definition as given in Formula I to produce a compound of Formula

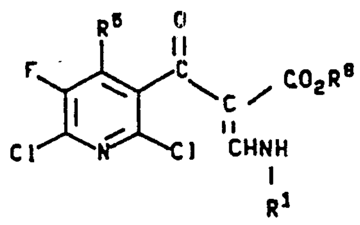

(c) cyclizing a compound of step (b) in the presence of a base to produce a compound of Formula II.